# FASCICULE DE BREVET EUROPEEN

(11) **EP 0 550 331 B1**
(45) Date de publication et mention de la délivrance du brevet: **28.08.1996**
(21) Numéro de dépôt: 92403541.3
(22) Date de dépôt: 23.12.1992
(51) Int. Cl.: B01J 23/18

(54) **Nouveau catalyseur d'isomérisation d'oléfines linéaires en oléfines branchées et utilisations de ce catalyseur, notamment pour l'isomérisation des normal-butènes en isobutène**
Neuer Katalysator für die Isomerisierung linearer Olefine zu verzweigten Olefinen und seine Verwendung, insbesondere für die Isomerisierung von n-Buten zu Isobuten
New catalyst for the isomerisation of linear olefins to branched olefins and use thereof, in particular for the isomerisation of n-butene to isobutene

(30) Priorité: 30.12.1991 FR 9116301
(43) Date de publication de la demande: 07.07.1993
(73) Titulaire: TOTAL RAFFINAGE DISTRIBUTION S.A., 92800 Puteaux (FR)
(72) Inventeur: Szabo, Georges, F-76290 Montivilliers (FR); Milan, Alain, F-76290 Montivilliers (FR)
(74) Mandataire: Jolly, Jean-Pierre

(56) Documents cités:
- WO-A-82/03186
- FR-A- 1 306 242
- FR-A- 2 376 103

## Description

La présente invention concerne un nouveau catalyseur d'isomérisation d'oléfines linéaires en oléfines branchées. L'invention concerne également l'utilisation de ce catalyseur dans des procédés d'isomérisation d'oléfines légères en oléfines branchées, notamment de n-butènes en isobutène.

C'est à cette application particulière du catalyseur onforme à l'invention que l'on se réfèrera plus spécifiquement ci-après, en raison de son importance économique, mais l'invention n'est pas limitée, bien entendu, à cette utilisation.

On sait que les additifs les plus couramment utilisés pour améliorer le pouvoir antidétonant des carburants pour moteurs à explosion sont des dérivés alkylés du plomb, notamment le plomb tétraéthyle.

Le développement, dans les automobiles, des pots d'échappement dits "catalytiques", qui utilisent comme catalyseur un métal noble tel que le platine, a toutefois amené les producteurs de carburant à substituer d'autres agents antidétonants aux dérivés de plomb, car ce métal est un poison bien connu des catalyseurs à base de métaux nobles.

Parmi les nouveaux additifs utilisés dans les carburants sans plomb, le méthyl tertio-butyl éther, ou MTBE, s'est révélé posséder des propriétés particulièrement intéressantes (voir, à ce sujet, FR-A- 2 484 800).

Ce MTBE est habituellement préparé en faisant réagir de l'isobutène avec du méthanol suivant la réaction réversible suivante :

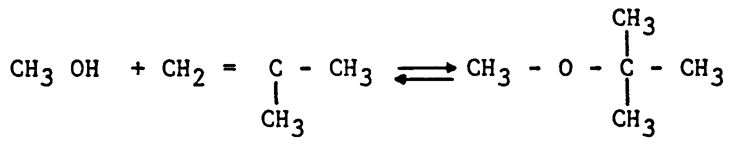

L'isobutène intervenant dans cette réaction provient notamment des coupes oléfiniques de raffinage du pétrole, en particulier des coupes C₄ issues du craquage catalytique, mais, en raison de l'équilibre thermodynamique, la quantité d'isobutène présent dans ces coupes est limitée et est généralement comprise entre 10 et 25 % en poids.

La quantité croissante d'isobutène utilisée pour la préparation du MTBE a donc amené les chercheurs à étudier des procédés d'isomérisation des butènes linéaires (butène-1 et butène-2 cis et trans) en isobutène (ou méthyl-2-propène). Alors que l'isomérisation catalytique entre les butènes linéaires est très aisée, la réaction d'isomérisation des n-butènes en isobutène, qui implique un déplacement d'un groupement méthyl et non le déplacement d'une double liaison oléfinique, est difficile à maîtriser, en raison de l'importance des réactions secondaires de polymérisation et de métathèse de l'isobutène.

Divers catalyseurs acides ont été proposés pour cette réaction d'isomérisation, notamment à base d'alumines ou de silices-alumines halogénées (voir US-A- 4 404 417).

Il a ensuite été établi que des catalyseurs à base de telles alumines ou silices-alumines chlorées ou fluorées et comportant certains additifs, dont un halogénure de bismuth, présentent une activité accrue (voir US-A- 4 405 500).

La présente invention concerne des catalyseurs de ce type, c'est-à-dire comprenant une alumine ou une silice-alumine halogénée et comportant du bismuth, et elle vise à optimiser à la fois la sélectivité, l'activité et la stabilité de tels catalyseurs.

Les catalyseurs de ce type présentent, en effet, l'inconvénient majeur, dans leur application à l'isomérisation de n-butènes en isobutène, de favoriser également des réactions secondaires, notamment de polymérisation, qui sont néfastes au rendement de la réaction d'isomérisation et qui affectent défavorablement l'activité du catalyseur, du fait du dépôt de polymères sur les sites actifs de celui-ci.

En étudiant ces catalyseurs, la Demanderesse a toutefois établi que, parmi les éléments du groupe V de la classification périodique présents sur ce catalyseur, certains agissent simultanément, mais de manière différente, sur l'activité et sur la sélectivité du catalyseur.

En effet, comme il ressortira des essais décrits ciaprès, si la sélectivité croît d'abord de façon sensiblement linéaire, en fonction de la teneur en bismuth, elle atteint très rapidement un palier, à partir duquel elle demeure sensiblement stable à partir d'une teneur aussi faible que 0, 10 % en poids, pour le bismuth.

Au contraire, l'activité relative du catalyseur activé au bismuth et éventuellement au phosphore ou à l'arsenic, c'est-à-dire le rapport de l'activité de ce catalyseur à celle du même support d'alumine ou de silice-alumine halogénée, sans additif, croît rapidement en fonction de la teneur en cet additif, passe par un maximum pour une teneur aussi faible qu'environ 0, 10 % en poids pour le bismuth, pour décroître ensuite et devenir inférieur à 1 à partir d'une teneur d'environ 0, 4 % en poids. On notera que la demande internationale WO-A-82 03 186 enseigne l'utilisation d'un catalyseur comprenant de 1 à 10%, de préférence environ 3%_{,} de bismuth, et que le brevet US-A-4 405 500 enseigne l'utilisation d'un catalyseur comprenant environ 9% en poids de bismuth.

L'invention a donc pour premier but d'optimiser l'activité de ce type de catalyseur, tout en conservant à celui-ci une sélectivité satisfaisante, dans son application à l'isomérisation des oléfines linéaires en oléfines branchées, notamment du butène-1 en isobutène.

La Demanderesse a par ailleurs établi qu'alors que les catalyseurs de la technique antérieure se désactivent rapidement en fonction du temps, lorsqu'on les utilise en présence d'hydrogène pour l'isomérisation d 'oléfines linéaires en oléfines branchées, l'activité des catalyseurs conformes à l'invention ne varie pratiquement pas au cours du temps, dans une telle utilisation.

Un autre but de l'invention est donc d'accroître la durée d'utilisation des catalyseurs à base d'alumine ou de silice-alumine halogénée contenant du bismuth et éventuellement du phosphore ou de l'arsenic, dans leur application à l'isomérisation d'oléfines linéaires en oléfines branchées, notamment de n-butènes en isobutène.

A cet effet, l'invention a pour premier objet un catalyseur d'isomérisation d'oléfines linéaires en oléfines branchées, notamment de n-butènes en isobutène, comprenant au moins un oxyde métallique réfractaire poreux choisi dans le groupe constitué par l'alumine, la silice, la silice alumine,la zircone et l'oxyde de titane, à la surface duquel sont présents au moins un halogène et du bismuth, ce catalyseur étant caractérisé en ce qu'il contient moins de 1 % en poids de bismuth et entre 0,01 et 2 % en poids de chlore.

De préférence, le catalyseur comprendra entre 0,1 et 0,8 % en poids de bismuth et entre 1,3 et 2 % en poids de chlore. En outre, il peut comprendre entre 0,01 % et 2 % en poids de phosphore et/ou d'arsenic.

Quand l'oxyde métallique réfractaire est constitué de silice alumine, il peut contenir jusqu'à 80 % de silice.

L'invention a également pour objet l'utilisation d'un tel catalyseur pour l'isomérisation d'oléfines linéaires en oléfines branchées, notamment de n-butènes en isobutène.

Les conditions d'isomérisation pourront être les suivantes :
- Température : comprise entre 20 et 550 °C et, de préférence, entre 300 et 500°C,
- Pression : entre 10⁵ et 2.10⁶ Pascals (entre 1 et 20 bars),
- Pression partielle de l'oléfine branchée : de 0,05 à 10 bars, de préférence entre 0,1 et 5 bars ;
- Débit liquide horaire : de 1 à 10 et, de préférence, de 1 à 5 volumes de charge liquide par volume de catalyseur et par heure (v.v.h.).

Pour réduire la pression partielle de l'oléfine branchée dans la charge et limiter ainsi les réactions de polymérisation, on diluera avantageusement la charge dans un gaz dans les conditions de la réaction, avec un rapport molaire gaz diluant/hydrocarbures oléfiniques compris entre 0,5 et 10.

Comme indiqué ci-dessus, dans une forme de mise en oeuvre particulièrement avantageuse du procédé d'isomérisation, une partie au moins du gaz diluant sera constituée par de l'hydrogène, lequel confère une activité stable en fonction du temps d'utilisation du catalyseur conforme à l'invention, à la différence des catalyseurs de la technique antérieure.

Le catalyseur conforme à l'invention pourra être préparé par des moyens usuels dans la technique.

L'oxyde réfractaire poreux utilisé pourra avoir une porosité comprise entre 10 et 500 m² /g et un rayon de pore moyen compris entre 10 et 500 Å (10⁻⁸ cm).

On l'imprégnera, par exemple, par une solution chlorhydrique d'un sel soluble du bismuth et, éventuellement, du phosphore et/ou de l'arsenic.

L'imprégnation est suivie par une évaporation complète de la solution, à l'évaporateur rotatif par exemple.

Cette imprégnation peut être effectuée industriellement en utilisant le volume minimum de solution correspondant au volume poreux du support. L'évaporateur n'est alors pas nécessaire.

Après cette imprègnation, le catalyseur est séché, puis calciné dans des conditions bien connues de l'homme de l'art.

D'autres méthodes peuvent être également utilisées.

Ainsi, les chlorures peuvent être déposés après le bismuth et, eventuellement, le phosphore et l'arsenic, par traitement du support les contenant, avec HCl gazeux par exemple. On obtient alors un catalyseur conforme à l'invention.

Les exemples qui suivent et qui n'ont pas de caractère limitatif illustrent la mise en oeuvre et les avantages de l'invention. Dans tous ces exemples, sauf pour le catalyseur B4, le support du catalyseur est une alumine gamma du commerce, ayant une surface spécifique de 185 m² /g et un volume poreux de 0,48 cm³ /g.

### EXEMPLE 1

On prépare un catalyseur témoin (catalyseur T) de la manière suivante :

50 g d'alumine, calcinée à 600° C, sont mis en contact avec 125 cm³ d'une solution obtenue en mélangeant 10 cm³ d'acide chlorhydrique R.P. avec 115 cm³ d'eau distillée.

La solution est évaporée sur l'alumine à l'évaporateur rotatif.

Le solide ainsi obtenu est ensuite séché pendant 16 heures à 120°C, puis calciné pendant 2 heures à 500°C.

Le catalyseur final est donc une alumine chlorée, contenant 1,78 % en poids de chlore, teneur déterminée par analyse par fluorescence X.

### EXEMPLE 2

On prépare des catalyseurs. B1, B2, B3 contenant du bismuth et du chlore. Dans ce but, 50g d'alumine sont mis en contact avec 125 cm³ d'une solution, obtenue par mélange de 115 cm³ d'eau distillée et de 10 cm³ d'acide chlorhydrique R.P., contenant également des quantités variables de nitrate de bismuth, Bi (NO₃)₃. 5H₂O.

Un quatrième catalyseur, B4, est préparé exactement de la même façon que B1, mais on remplace le support d'alumine par 50 g d'un support de silice-alumine, contenant 20 % de silice et ayant 210 m²/g de surface spécifique et un volume poreux de 0,52 cm³/g.

Les quantités de nitrate de bismuth utilisées pour les différentes préparation sont :

| Catalyseur | Nitrate de Bi (grammes) |
|---|---|
| T (référence) | - |
| B1 | 0,116 |
| B2 | 0,58 |
| B3 | 1,16 |
| B4 | 0,116 |

Les teneurs en chlore et bismuth sont données dans le Tableau I ci-après :

**TABLEAU I**

| Composition des catalyseurs | | |
|---|---|---|
| CATALYSEUR | % Cl | % Bi |
| T | 1,76 | 0 |
| B1 | 1,78 | 0,1 |
| B2 | 1,71 | 0,5 |
| B3 | 1,51 | 1,0 |
| B4 | 1,6 | 0,1 |

### EXEMPLE 3

On prépare un catalyseur contenant du bismuth, du phosphore et du chlore.

50g d'alumine sont mis en contact avec 125 cm³ d'une solution obtenue par mélange de 115 cm³ d'eau distillée et de 10 cm³ d'acide chlorhydrique R.P., contenant également 0,186 g de dihydrogénophosphate d'ammonium NH₄ H₂ PO₄ et 0,116 g de nitrate de bismuth.

On obtient ainsi un catalyseur B5 contenant 1,60% de chlore, 0,1 % de bismuth et 0,1% de phosphore.

### EXEMPLE 4

Dans cet exemple, on se propose d'étudier les propriétés catalytiques des catalyseurs préparés dans les exemples précédents, en les utilisant dans des tests d'isomérisation du butène-1.

Ces tests sont effectués sous la pression atmosphérique, avec 4 g de catalyseur et avec dilution de la charge de butène-1 par de l'hydrogène. Le taux de dilution, dans tous les cas, est de 10 moles d'hydrogène diluant pour 1 mole de butène-1.

Le Tableau II, ci-après rassemble les différentes conditions d'essais et les résultats obtenus.

Ce Tableau II compare les propriétés des catalyseurs pour un même niveau d'activité (85 % de conversion du butène), dans des conditions identiques, mais avec des débits variables de la charge.

**TABLEAU II**

| Propriétés catalytiques des catalyseurs dans les conditions d'utilisation : T = 450 °C ; P=1 bar; rapport Hydrogène/butène-1 = 10/1 molaire ; débit de charge variable, de façon à obtenir 85 % de conversion du butène-1. | | | | | |
|---|---|---|---|---|---|
| Catalyseur | Diluant | Débit de Charge g/g cat/h | Rendements (% en poids) | | |
| | | | Isobutène | Butanes | Produits de métathèse |
| T | Hydrogène | 2,0 | 34,08 | 6,36 | 2,47 |
| B1 | Hydrogène | 2,2 | 35,01 | 5,45 | 2,56 |
| B2 | " | 1,9 | 34,99 | 5,14 | 2,47 |
| B3 | " | 1,7 | 35,24 | 4,75 | 2,76 |
| B4 | " | 2,3 | 35,00 | 5,50 | 2,60 |
| B5 | " | 1,5 | 35,28 | 5,17 | 2,28 |

Les résultats du Tableau II montrent que la sélectivité des catalyseurs contenant du bismuth et éventuellement du phosphore est plus élevée que celle du catalyseur témoin.

D'autre part, l'activité du catalyseur B1 contenant 0,1 % de Bi est plus élevée que celle n'en contenant pas (même activité observée pour un débit de charge plus élevé).

Les rendements en butanes diminuent quand les teneurs en bismuth augmentent.

On remarquera enfin que le remplacement du support d'alumine par un support d'alumine-silice (catalyseur B4) conduit à un catalyseur ayant des propriétés pratiquement inchangées.

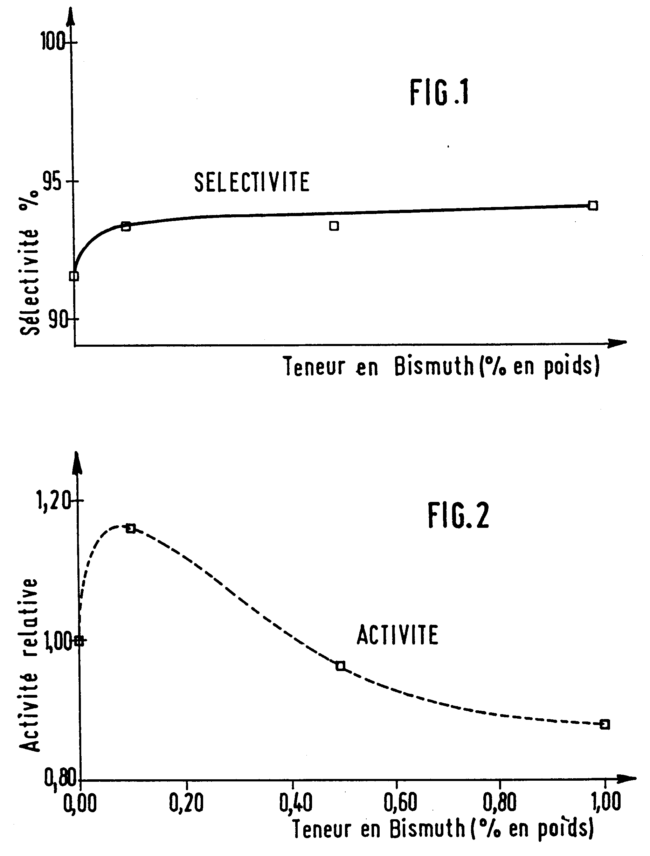

L'effet de la teneur en bismuth sur l'activité et la sélectivité est illustrée par les figures 1 et 2, des dessins annexés. Sur ces dessins :
- la figure 1 illustre la variation de la sélectivité des catalyseurs, exprimée en %, en fonction de la teneur en bismuth de ces catalyseurs, exprimée en % en poids.
- la figure 2 illustre la variation de l'activité relative du catalyseur (rapport de l'activité des catalyseurs contenant du bismuth à l'activité du catalyseur témoin), dans l'isomérisation du butène 1, en fonction de la teneur en bismuth de catalyseur exprimée en % en poids ;

On constate que l'activité croit très rapidement avec le teneur en bismuth, passe par un maximum pour des teneurs très faibles en bismuth et décroît ensuite.

La séléctivité, elle, croît d'abord assez rapidement avec la teneur en bismuth, puis continue à croître beaucoup plus lentement.

### EXEMPLE 5

Cet exemple est destiné à démontrer la remarquable stabilité du catalyseur B1 en présence d'hydrogène.

Il s'agit de résultats de tests pilote en continu, utilisant 20 cm³ de catalyseur, du butène-1 comme charge, dilué avec 10 molécules de gaz diluant (azote ou hydrogène) pour une molécule d'oléfine.

Le Tableau III compare la stabilité au cours de deux essais, l'un en présence d'hydrogène, l'autre en présence d'azote.

**TABLEAU III**

| Variation au cours du temps de l'activité du catalyseur B1. Conditions d'utilisation : Température : 450°C ; Pression totale : 5 bars; Débit de la charge : 3 g/g/h. | | |
|---|---|---|
| Temps en heures | Diluant : Hydrogène | Diluant : Azote |
| | % Conversion du butène-1 | % Conversion du butène-1 |
| 1 | | 79,9 |
| 5 | 83,7 | |
| 29 | 82,95 | |
| 39 | | 79,1 |
| 50 | | 78,5 |
| 60,2 | 83,6 | |
| 80 | | 76,2 |
| 100 | | 77,7 |
| 107 | 83,5 | |
| 120 | | 77 |
| 131,4 | 82,8 | |
| 150 | | 76,6 |
| 158,6 | 82,8 | |
| 180 | | 76 |
| 200 | | 75,4 |
| 204 | 83,9 | |
| 235,25 | 83,5 | |
| 269,1 | 83,2 | |
| 291,33 | 83,1 | |

Ce Tableau V montre qu'en présence d'hydrogène, le catalyseur B1 présente une activité qui ne diminue pas au cours du temps, à l'inverse du test avec l'azote comme diluant.

## Revendications

1. Catalyseur d'isomérisation d'oléfines linéaires en oléfines branchées, notamment de n-butènes en isobutène, comprenant au moins un oxyde métallique réfractaire poreux choisi dans le groupe constitué par l'alumine, la silice, la silice alumine, la zircone et l'oxyde de titane, à la surface duquel sont présents au moins un halogène et du bismuth, ce catalyseur étant caractérisé en ce qu'il contient respectivement moins de 1 % en poids de bismuth, et entre 0,01 et 2 % en poids de chlore.

2. Catalyseur selon la revendication 1, caractérisé en ce qu'il comprend entre 1,3 et 2 % en poids de chlore et entre 0,1 et 0,8 % en poids de bismuth.

3. Catalyseur selon l'une des revendications 1 et 2, caractérisé en ce qu'en plus du bismuth, il comprend entre 0,01 et 2 % en poids de phosphore et/ou d'arsenic.

4. Catalyseur selon l'une des revendications 1 à 3, caractérisé en ce que l'oxyde réfractaire contient au moins 20 % d'alumine lorsqu'il s'agit de silice alumine.

5. Catalyseur selon l'une des revendication 1 à 4, caractérisé en ce que le dépôt desdits éléments est obtenu par imprégnation de l'oxyde métallique réfractaire poreux par des sels des dits éléments et par leur décomposition thermique.

6. Catalyseur selon l'une des revendications 1 à 5, caractérisé en ce que l'oxyde réfractaire poreux a une porosité comprise entre 10 et 500 m²/g et un rayon de pore moyen compris entre 10 et 500 A (10⁻⁸ cm).

7. Utilisation d'un catalyseur selon l'une des revendications 1 à 6 pour l'isomérisation d'oléfines linéaires en oléfines branchées, notamment de n-butènes en isobutène.

8. Utilisation selon la revendication 7, caractérisée par les conditions réactionnelles suivantes :
- Température : comprise entre 100 et 550 °C et, de préférence, entre 300 et 500°C,
- Pression : entre 10⁵ et 2.10⁶ Pascals (entre 1 et 20 bars),
- Pression partielle de l'oléfine branchée : entre 0,05 et 10 bars, de préférence entre 0,1 et 5 bars ;
- Débit liquide horaire : de 1 à 10 et, de préférence, de 1 à 5 volumes de charge liquide par volume de catalyseur et par heure.

9. Utilisation selon l'une des revendications 7 et 8, caractérisée en ce que l'oléfine branchée est diluée dans un gaz ou un mélange de gaz dans les conditions de la réaction, avec un rapport molaire dudit gaz (ou dudit mélange) gaz diluant/hydrocarbure oléfinique compris entre 0,2 et 10.

10. Utilisation selon la revendication 9, caractérisé en ce que ledit gaz contient au moins 10 % en moles d'hydrogène.

## Claims

1. A catalyst for isomerising linear olefins into branched olefins, in particular n-butenes into isobutene, comprising at least one porous refractory metal oxide selected from the group consisting of alumina, silica, alumina/silica, zirconia and titanium oxide, on the surface of which there are present bismuth and at least one halogen, this catalyst being characterised in that it contains respectively less than 1 % by weight of bismuth and between 0.01 and 2 % by weight of chlorine.

2. A catalyst according to claim 1, characterised in that it comprises between 1.3 and 2 % by weight of chlorine and between 0.1 and 0.8 % by weight of bismuth.

3. A catalyst according to either one of claims 1 and 2, characterised in that, in addition to bismuth, it comprises between 0.01 and 2 % by weight of phosphorus and/or arsenic.

4. A catalyst according to any one of claims 1 to 3, characterised in that the refractory oxide contains at least 20 % of alumina when the latter is alumina/silica.

5. A catalyst according to any one of claims 1 to 4, characterised in that deposition of said elements is effected by impregnation of the porous refractory metal oxide by salts of said elements and by their thermal decomposition.

6. A catalyst according to any one of claims 1 to 5, characterised in that the porous refractory oxide has a porosity of between 10 and 500 m²/g and an average pore radius of between 10 and 500 A (10⁻⁸ cm).

7. Use of a catalyst according to any one of claims 1 to 6 for isomerising linear olefins into branched olefins, in particular n-butenes into isobutene.

8. Use according to claim 7, characterised by the following reaction conditions:
- temperature: between 100 and 550 °C and, preferably, between 300 and 500 °C,
- pressure: between 10⁵ and 2.10⁶ Pascals (between 1 and 20 bar),
- partial pressure of the branched olefin: between 0.05 and 10 bar, preferably between 0.1 and 5 bar;
- hourly liquid output: from 1 to 10 and, preferably, from 1 to 5 volumes of liquid load per volume of catalyst and per hour.

9. Use according to either one of claims 7 and 8, characterised in that the branched olefin is diluted in a gas or a mixture of gas under the reaction conditions, with a mole ratio of said diluting gas (or said mixture)/olefinic hydrocarbon of between 0.2 and 10.

10. Use according to claim 9, characterised in that said gas contains at least 10 % of hydrogen moles.

## Patentansprüche

1. Katalysator für die Isomerisation linearer Olefine zu verzweigten Olefinen, insbesondere von n-Butenen zu Isobuten, welcher mindestens ein poröses feuerfestes Metalloxid aus der Gruppe bestehend aus Aluminiumoxid, Siliciumoxid, Siliciumoxid-Aluminiumoxid, Zirkonoxid und Titanoxid aufweist, an dessen Oberfläche mindestens ein Halogen und Wismut vorliegen, dadurch **gekennzeichnet,** daß er weniger als 1 Gewichtsprozent Wismut und zwischen 0,01 und 2 Gewichtsprozent Chlor enthält.

2. Katalysator nach Anspruch 1, dadurch **gekennzeichnet,** daß er zwischen 1,3 und 2 Gewichtsprozent Chlor und zwischen 0,1 und 0,8 Gewichtsprozent Wismut aufweist.

3. Katalysator nach Anspruch 1 oder 2, dadurch **gekennzeichnet,** daß er zusätzlich zum Wismut zwischen 0,01 und 2 Gewichtsprozent Phosphor und/oder Arsen aufweist.

4. Katalysator nach Anspruch 1, 2 oder 3, dadurch **gekennzeichnet,** daß das feuerfeste Oxid mindestens 20 % Aluminiumoxid enthält, wenn es sich um Siliciumoxid-Aluminiumoxid handelt.

5. Katalysator nach einem der Ansprüche 1 bis 4, dadurch **gekennzeichnet,** daß die Ablagerung der besagten Elemente durch Imprägnierung des porösen feuerfesten Metalloxids mit Salzen der Elemente und durch deren thermische Zersetzung erhalten wird.

6. Katalysator nach einem der Ansprüche 1 bis 5, dadurch **gekennzeichnet,** daß das poröse feuerfeste Oxid eine Porosität zwischen 10 und 500 m²/g und einen mittleren Porenradius zwischen 10 und 500 A ( 10⁻⁸ cm) aufweist.

7. Verwendung eines Katalysators nach einem der Ansprüche 1 bis 6 für die Isomerisation linearer Olefine zu verzweigten Olefinen, insbesondere von n-Butenen zu Isobuten.

8. Verwendung nach Anspruch 7, **gekennzeichnet** durch die folgenden Reaktionsbedingungen:
Temperatur: zwischen 100 und 550°C, vorzugsweise zwischen 300 und 500°C,
Druck: zwischen 10⁵ und 2·10⁶ Pascal (zwischen 1 und 20 bar),
Partialdruck des verzweigten Olefins: zwischen 0,05 und 10 bar, vorzugsweise zwischen 0,1 und 5 bar,
stündlicher flüssiger Durchsatz: von 1 bis 10, vorzugsweise von 1 bis 5 Volumen flüssiger Charge pro Katalysatorvolumen und pro Stunde.

9. Verwendung nach Anspruch 7 oder 8, dadurch **gekennzeichnet,** daß das verzweigte Olefin in einem Gas oder einem Gasgemisch unter den Bedingungen der Reaktion verdünnt ist, und zwar mit einem Molverhältnis des verdünnenden Gases ( bzw. Gasgemisches ) zum olefinischen Kohlenwasserstoff zwischen 0,2 und 10.

10. Verwendung nach Anspruch 9, dadurch **gekennzeichnet,** daß das besagte Gas mindestens 10 Molprozent Wasserstoff enthält.
